# EUROPÄISCHE PATENTANMELDUNG

(11) **EP 2 913 050 A1**
(43) Veröffentlichungstag der Anmeldung: **02.09.2015**
(21) Anmeldenummer: 14157249.5
(22) Anmeldetag: 28.02.2014
(51) Int. Cl.: A61K 31/02, A61K 31/08, C08B 37/16, A61P 23/00

(54) **Verfahren zur Herstellung eines Flurankomplexes**

(71) Anmelder: SapioTec GmbH, 97082 Würzburg (DE)
(72) Erfinder: Die Erfindernennung liegt noch nicht vor
(74) Vertreter: Glawe, Delfs, Moll

(57) **Zusammenfassung**

Die Erfindung betrifft ein Verfahren zur Herstellung eines Flurankomplexes, folgenden Schritten: Herstellen einer wässrigen Lösung von Sulfobutylether-β-Cyclodextrin (SBECD); Temperieren dieser Lösung auf eine Temperatur von 2 bis 15°C; Zugabe des Flurans zur der wässrigen Lösung; Reagierenlassen zur Herstellung des Komplexes; und Abtrennen des Komplexes.

## Beschreibung

Die Erfindung betrifft einen Flurankomplex sowie dessen Formulierung als Anästhetikum.

Flurane sind Arzneistoffe, die in der Anästhesie zur Aufrechterhaltung oder Einleitung der Narkose eingesetzt werden und im Stand der Technik durch Inhalation aufgenommen werden. Inhalationsanästhetika werden als Gase oder mittels eines Vaporisers verdampfte Flüssigkeiten über eine Atemmaske, eine Larynxmaske oder einen Endotrachealtubus appliziert.

Flurane weisen einen niedrigen Siedepunkt und hohen Dampfdruck auf. Es handelt sich um mehrfach halogenierte Ether. Es handelt sich um lipophile Stoffe, deren anästhetische Wirksamkeit unter anderem mit einer aus der Lipophilie herrührenden unspezifischen Interaktion mit Bestandteilen der Zellmembran erklärt wird.

Es ist bereits bekannt, Flurane mit Cyclodextrinen zu komplexieren (WO 2011/086146 A1, DE 10 2010 042 615 A1). Auf diese Weise sollen diese flüchtigen Stoffe lagerstabil und problemlos applizierbar bereitgestellt werden. Sowohl die Lagerstabilität als auch insbesondere die Formulierung zu einer in vivo administrierbaren Form sind jedoch nach wie vor problematisch.

Der Erfindung liegt die Aufgabe zu Grunde, ein Verfahren der eingangs genannten Art zu schaffen, mit dem eine effiziente und unproblematische Herstellung einer lagerfähigen und problemlos administrierbaren Form von Fluranen möglich ist.

Gelöst wird diese Aufgabe durch ein Verfahren mit den folgenden Schritten:
a. Herstellen einer wässrigen Lösung von Sulfobutylether-β-Cyclodextrin (SBECD),
b. Temperieren dieser Lösung auf eine Temperatur von 2 bis 15°C,
c. Zugabe des Flurans zur der wässrigen Lösung,
d. Reagierenlassen zur Herstellung des Komplexes,
e. Abtrennen des Komplexes.

Die Erfindung hat erkannt, dass sich überraschenderweise durch die Kombination der Auswahl von SBECD und die Komplexierung bei der beanspruchten tiefen Temperatur von 2-15°C ein Komplex herstellen lässt, der einerseits ohne weiteres lagerfähig ist, ohne das flüchtige Fluran freizusetzen und andererseits völlig unproblematisch in eine administrierbare Form gebracht werden kann, beispielsweise in Wasser gelöst werden kann, ohne dass es dazu besonderer Maßnahmen oder Hilfsmittel bedarf.

Cyclodextrine weisen generell eine Toroidform auf und besitzen eine entsprechend geformte Kavität. Die Flurane treten als Gastmolekül in diese Kavität ein, so dass ein in wässriger Lösung formulierbarer Komplex der extrem lipophilen Flurane erhalten wird, der die Flurane am vorgesehenen pharmazeutischen Wirkort zur Verfügung stellen kann.

Cyclodextrine sind zyklische Oligosaccharide aus α-1,4-glykosidisch verknüpften Glukosemolekülen. ß-Cyclodextrin besitzt sieben Glukoseeinheiten. Bei einem Sulfobutylether-ß-Cyclodextrin sind Hydroxygruppen der Glukoseeinheit in einem Sulfobutylalkohol verethert. Erfindungsgemäß ist in der Regel lediglich ein Teil der 21 Hydroxygruppen eines β-Cyclodextrins verethert.

Die Herstellung von Sulfoalkyethercyclodextrinen ist dem Fachmann geläufig und beispielsweise in US 5,134,127 oder WO 2009/134347 A2 beschrieben.

Sulfobutylethergruppen werden bei Cyclodextrinen im Stand der Technik zur Erhöhung der Hydrophilie beziehungsweise Wasserlöslichkeit eingesetzt. Die Erfindung hat erkannt, dass die Sulfobutylethergruppen in besonderem Maße zur Erhöhung der Stabilität des Komplexes aus Fluranen und dementsprechend substituierten ß-Cyclodextrin beitragen und so die Lagerstabilität und Formulierbarkeit der besonders flüchtigen Flurane wesentlich verbessern. Der erfindungsgemäß erhältliche Komplex kann als lagerstabile wässrige Lösung oder Feststoff formuliert werden, wie unten noch näher gezeigt werden wird.

Bevorzugt beträgt der Substitutionsgrad des Cyclodextrins mit Sulfobutylethergruppen 3 bis 8, weitervorzugsweise 4 bis 7. Geeignete Sulfobutylether-ß-Cyclodextrine mit einem mittlerem Substitutionsgrad von 6 bis 7 sind beispielsweise in der genannten WO 2009/134347 A2 beschrieben und unter dem Handelsnamen Captisol® kommerziell erhältlich. Ebenfalls verwendbar sind entsprechende Cyclodextrine mit einem Substitutionsgrad von 4-5, beispielsweise 4,2.

Das Fluran ist ein mehrfach flourierter Ether und bevorzugt ausgewählt aus der Gruppe bestehend aus Sevofluran, Enfluran, Isofluran, Desfluran und Methoxyfluran. Sevofluran ist besonders bevorzugt.

Die WO 2011/086146 A1 offenbart zwar ebenfalls die Herstellung eines lagerstabilen Komplexes (mit α-Cyclodextrinen), jedoch hat sich gezeigt, dass diese Komplexe nicht mehr ohne weiteres in wässrige Lösung gebracht werden können, sondern zu diesem Zweck besondere Maßnahmen oder Hilfsstoffe erforderlich sind. Die Formulierung beispielsweise einer i.v. administrierbaren Lösung nach diesem Stand der Technik ist somit problematisch. Die DE 10 2010 042 615 A1 offenbart eine Komplexierung von Fluranen mit verschiedenen Cyclodextrinen, darunter auch SBECD, die technische Lehre ist jedoch eine Komplexierung bei hohen Temperaturen von 50°C. Diese hohe Temperatur wird für eine erfolgreiche Komplexierung für erforderlich gehalten. Hingegen hat die vorliegende Erfindung erkannt, dass eine Komplexierung bei sehr niedrigen Temperaturen überraschenderweise möglich ist und zudem den erheblichen Vorteil hat, dass es nicht oder in sehr viel geringerem Maße zu einer Freisetzung von gasförmigen Fluranen kommt, deren Siedepunkte bei oder unter 50°C liegen.

Das Reagierenlassen zur Herstellung des Komplexes erfolgt bevorzugt in einem geschlossenen Behälter unter Luftabschluss. Bevorzugt ist ein Zeitraum von 1 bis 10 h, weiter bevorzugt 2 bis 5 h, beispielsweise etwa 3 h. Bevorzugt wird gerührt oder auf eine sonstige geeignete Weise gemischt. Nach Abschluss dieser Reaktion erhält man bevorzugt eine homogene, flüssige Phase.

Das Abtrennen des Komplexes kann ein teilweises oder vollständiges Entfernen des Lösungsmittels Wasser umfassen.

Erfindungsgemäß enthält bevorzugt die in Schritt a. hergestellte wässrige Lösung SBECD in einer Konzentration von 5 bis 20 Gew.-%, vorzugsweise 10 bis 15 Gew.-%. In diesem Konzentrationsbereich ergibt sich in Kombination mit dem beanspruchten Temperaturbereich eine weitgehend vollständige Komplexierung, der entstandene Komplex bleibt in homogener, einphasiger wässriger Lösung.

Die Zugabe des Flurans in Schritt c. erfolgt bevorzugt in einem Molverhältnis zum SBECD von 4:1 bis 1:1, weiter vorzugsweise 3:1 bis 2:1.

Im Rahmen der Erfindung kann die hergestellte wässrige Lösung des Komplexes, gegebenenfalls nach dem Abziehen eines Teil des Lösungsmittels Wasser, unmittelbar Verwendung finden, beispielsweise als i.v. administrierbare Lösung. Bevorzugt wird jedoch der erhaltene Komplex als Feststoff abgetrennt, dies kann erfindungsgemäß besonders bevorzugt erfolgen durch ein Abziehen des Lösungsmittels durch Gefriertrocknung (Lyophilisation). Besonders bevorzugte Bedingungen für die Gefriertrocknung sind ein Zeitraum von 12 bis 48 h, beispielsweise etwa 24 h; eine Temperatur von -30 bis -70 °C, beispielsweise etwa -50 °C; sowie ein Vakuum, beispielsweise ein Druck von 1 bis 20 Pa, vorzugsweise 5 bis 10 Pa. Man erhält so einen Komplex, in dem die chemische Struktur und das amorphe Erscheinungsbild des Komplexbildners im wesentlichen unverändert erhalten ist und der einen geringen Restwassergehalt von typischerweise unter 5 Gew.-%, beispielsweise etwa 3-4 Gew.-%, aufweist.

Gegenstand der Erfindung ist ferner ein durch das erfindungsgemäße Verfahren erhältlicher Komplex. Der Flurangehalt dieses Komplexes ist erfindungsgemäß bevorzugt größer als 8 Gew.-%, weiter vorzugsweise größer als 8,2 Gew.-%, weiter vorzugsweise größer als 8,5 Gew.-%. Besonders bevorzugt ist in dem Komplex das komplexierte Fluran Sevofluran.

Das Molverhältnis von SBECD zu Fluran beträgt in dem erfindungsgemäßen Komplex bevorzugt wenigstens 1:0,6, weiter vorzugsweise wenigstens 1:0,7, weiter vorzugsweise wenigstens 1:0,8, weiter vorzugsweise wenigstens 1:0,85. Der erfindungsgemäß erhältliche Komplex weist somit einen sehr hohen molaren Gehalt des Flurans bei gleichzeitig hoher Lagerstabilität und problemloser Formulierbarkeit zu einer administrierbaren Form, beispielsweise einer wässrigen Lösung, auf.

Gegenstand der Erfindung ist ferner ein erfindungsgemäß erhältlicher Komplex zur Verwendung als Medikament. Ein weiterer Gegenstand der Erfindung ist ein für orale und/oder intravenöse Administration formuliertes Anästhetikum, das einen erfindungsgemäß erhältlichen Komplex aufweist.

Ausführungsbeispiele der Erfindung werden nachfolgend beschrieben. In den Zeichnungen zeigen:
- Fig. 1:: Röntgenpulverdiffraktogramme von SBECD und einem erfindungsgemäßen Komplex;
- Fig. 2:: Kapillarelektropherogramme von SBECD und einem erfindungsgemäßen Komplex;

1. Verwendete Materialien
   - destilliertes Wasser
   - Sulfobutylether-β-Cyclodextrin (SBECD)
   - Sevofluran
   SBECD wurde bezogen von der Firma Cyclo Lab Cyclodextrin Research & Development Laboratory Ltd., Ungarn. Sevofluran wurde bezogen von Abbott GmbH, Wiesbaden.
2. Bestimmung des Sevofluran-Gehaltes hergestellter Komplexe
   Die Bestimmung des Sevofluran-Gehaltes der Komplexe erfolgte gaschromatografisch. Die Gaschromatografiebedingungen waren wie folgt:
   - Gaschromatograph:: Shimadzu GC-17A
   - Detector: Injector:: Flame ionization detector (FID) Shimadzu AoC-5000 auto injector
   - *Software:*: Shimadzu Class-VP 7.4 Version
   - *Gase:*:
   - Träger:: Helium (99.999 %)
   - weitere Gase:: Stickstoff (99.999%)
   synthetische Luft (99.999%)
   Wasserstoff (Whatman Hydrogen generator)
   - Säule:: Rtx624 (30 m x 0.32 mm x 1.8 mm) (Restek)

### TEMPERATURPROGRAMM:

| Rate (°C/min) | Temperatur (°C) : | Zeit (min) : |
|---|---|---|
| - | 38 | 4.0 |
| 40 | 220 | 1.5 |

| | |
|---|---|
| *Injektortemperatur:* | 220°C |
| *Detektortemperatur:* | 220°C |
| *Split ratio:* | 100:1 |
| *Geschwindigkeit:* | 30 cm/s |

Das Injektionsprogramm war wie folgt: Nach einer Inkubationszeit von 10 min bei 60°C wurde eine Dampfprobe mit einem Volumen von 250 µl bei 70 °C in den Gaschromatografen injiziert.

### Probenvorbereitung

Vergleichslösungen: 1 ml destilliertes Wasser mit 250 µl DMF wird in die Viale gegeben.

Kalibrierlösungen: Als Stammlösung wird 100 mg Sevofluran in 2 ml Glasflaschen eingewogen und bis zur Markierung mit DMF aufgefüllt.

Verschiedene Mengen dieser Stammlösung (20, 65, 110, 155 und 200 µl) werden jeweils mit DMF auf 200 µl aufgefüllt und mit 1 ml destilliertem Wasser in Head Space Vials (19,5 ml) eingefüllt.

Probenlösung: 50 mg des Probenkomplexes wird in eine Head Space Vial (19,5 ml) gegeben mit 1 ml destilliertem Wasser und 200 µl DMF.

### Beispiel 1

Dieses Beispiel beschreibt die Herstellung eines SBECD-Komplexes von Sevofluran.

In einem Rundkolben wurden unter fortlaufendem Rühren bei Raumtemperatur 22,8 g (10,54 mmol) SBECD in 190 ml Wasser gelöst. Es entstand eine klare Lösung, die auf 8 °C abgekühlt wurde. 5,1 g (25 mmol) Sevofluran wurden hinzugefügt, der Rundkolben wurde fest verschlossen und es wurde bei 8 °C über einen Zeitraum von 3 h mit 400 min⁻¹ gerührt. Man erhielt eine homogene flüssige Phase mit einem Sevofluran-gehalt von 0,75 Gew.-%.

Die erhaltene Lösung wurde auf -50 °C eingefroren und für 24 h bei einem Druck im Bereich 6.0 bis 8.7 Pa lyophilisiert. Man erhielt einen amorphen Feststoff, der gemahlen und durch ein Sieb mit einer Maschenweite von 0,3 mm gesiebt wurde. Der Restgehalt an Wasser war gering (Daten siehe weiter unten), so dass kein weiterer Gefriertrocknungsschritt erforderlich war.

Der Gehalt des festen Komplexes an Sevofluran (Bestimmung wie oben beschrieben) betrug 8,6 Gew.-%.

### Beispiel 2

In diesem Beispiel wurde untersucht, welchen Einfluss die Temperatur bei der Herstellung auf den Komplex hat. Die Herstellung des Beispiels 1 wurde wiederholt mit Abweichungen bei der Temperatur der Lösung bei der Herstellung (nicht erfindungsgemäße Vergleichsbeispiele).

Bei 22°C Herstellungstemperatur betrug der Gehalt des festen Komplexes an Sevofluran 1,7 Gew.-%, bei 30°C 0,7 Gew.-%.

Man erkennt, dass bereits bei Temperaturen von 22 °C, insbesondere auch 30 °C, lediglich eine ganz geringe Ausbeute erzielt werden kann. Der Sevofluran-Gehalt des Komplexes ist signifikant geringer als im erfindungsgemäßen Verfahren. Das im Stand der Technik gemäß DE 10 2010 042 615 A1 offenbarte Verfahren (Herstellung bei 50 °C) dürfte daher praktisch nicht durchführbar sein.

### Beispiel 3

Die Struktur von SBECD und dem Komplex gemäß Beispiel 1 wurde mittels Röntgendiffrakrometrie überprüft. Es wurde übliche CuKα-Strahlung verwendet. Die Reflexionspeaks wurden in einem Bereich des Winkels 2θ von 5 bis 40° aufgezeichnet.

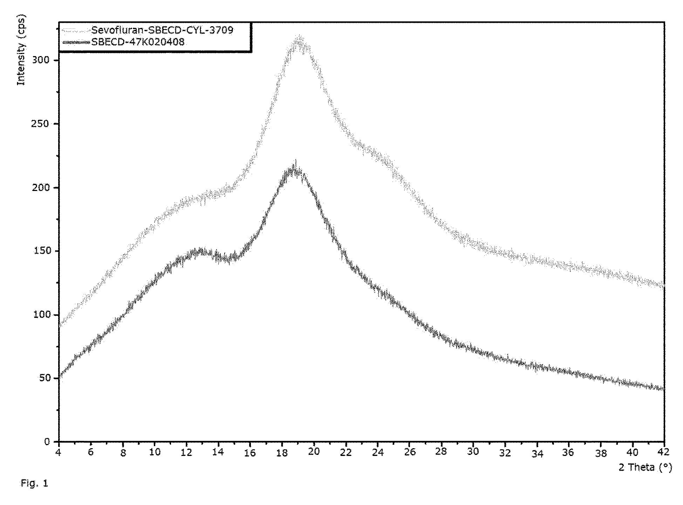

In Fig. 1 ist zu erkennen, dass SBECD (untere Kurve) und der Komplex (obere Kurve) gleichermaßen das Reflektionsmuster eines amorphen Stoffes aufweisen.

Die amorphe Struktur wurde bestätigt durch Beobachtung unter einem Lichtmikroskop bei 100facher Vergrößerung unter polarisiertem Licht. Es waren keine Interferenzmuster sichtbar, dies bestätigt die amorphe Struktur.

### Beispiel 4

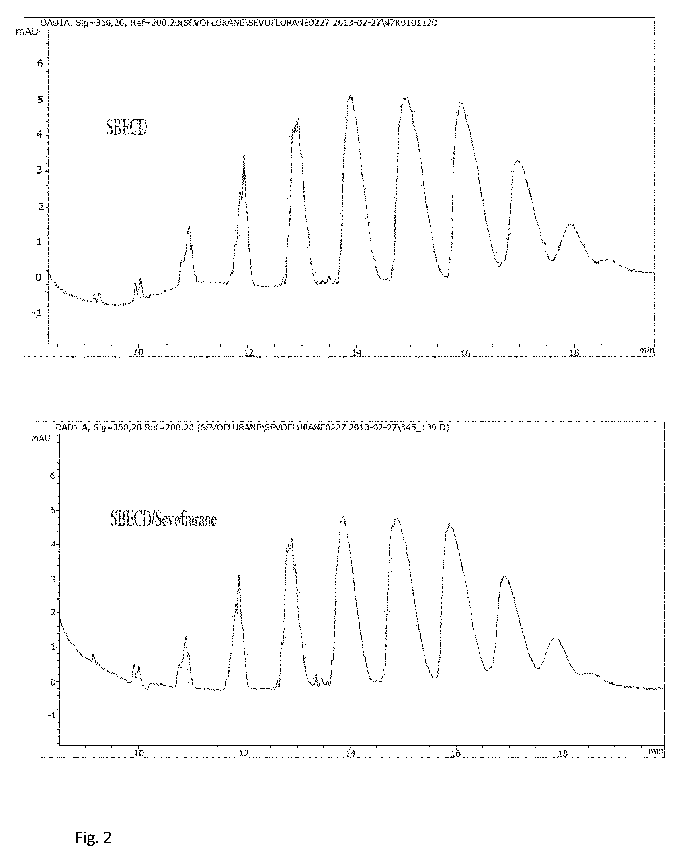

In diesem Beispiel wurde durch Kapillarelektrophorese bestätigt, dass die Struktur des Komplexbildners durch das erfindungsgemäße Verfahren nicht beeinträchtigt wird und intakt bleibt. Fig. 2 zeigt (oben) das Elektropherogramm von SBECD und unten das entsprechende Elektropherogramm des Komplexes gemäß Beispiel 1. Der Vergleich zeigt, dass die Struktur des Komplexbildners durch das erfindungsgemäße Verfahren nicht beeinträchtigt wird.

### Beispiel 5

Der gemäß Beispiel 1 erhaltene Feststoff wurde 14 Tage (14 d) in einem offenen Behälter bei 40 °C gelagert. In der nachfolgenden Tabelle aufgeführte Eigenschaften wurden in den angegebenen Zeitabständen bestimmt.

| | 0 d | 2 d | 7 d | 14 d |
|---|---|---|---|---|
| Sevofluran-Gehalt [Gew.-%] | 8,6 | 8,6 | 8,6 | 8,6 |
| Wiederlösbarkeit | klar | | | klar |
| Restwassergehalt [Gew.-%] | 3,5 | | | 4,7 |

Bei der Ermittlung der Wiederlösbarkeit wurden 100 mg des Komplexes in 0,9 ml destilliertem Wasser dispergiert. Die Bestimmung des Restwassergehaltes wurde nach Karl-Fischer durchgeführt. Beide Parameter wurden nur zu Beginn des Versuches und zum Abschluss nach 14 Tagen ermittelt.

Man erkennt, dass sich der Sevofluran-Gehalt auch bei längerer Lagerung unter ungünstigen Bedingungen nicht ändert, der Komplex somit sehr stabil ist. Er lässt sich auch nach 14 Tagen unter diesen Lagerbedingungen problemlos mit Wasser und ohne weitere Hilfsmittel wieder zu einer klaren Lösung ansetzen. Der Restwassergehalt war unter diesen ungünstigen Lagerbedingungen nur geringfügig angestiegen.

## Patentansprüche

1. Verfahren zur Herstellung eines Flurankomplexes, **gekennzeichnet durch** folgende Schritte:
a. Herstellen einer wässrigen Lösung von Sulfobutylether-β-Cyclodextrin (SBECD),
b. Temperieren dieser Lösung auf eine Temperatur von 2 bis 15°C,
c. Zugabe des Flurans zur der wässrigen Lösung,
d. Reagierenlassen zur Herstellung des Komplexes,
e. Abtrennen des Komplexes.

2. Verfahren nach Anspruch 1, **dadurch gekennzeichnet, dass** die in Schritt a. hergestellte wässrige Lösung SBECD in einer Konzentration von 5 bis 20 Gew.-%, vorzugsweise 10 bis 15 Gew.-% enthält.

3. Verfahren nach Anspruch 1 oder 2, **dadurch gekennzeichnet, dass** die Zugabe des Flurans in Schritt c. in einem Molverhältnis zum SBECD von 4:1 bis 1:1, vorzugsweise 3:1 bis 2:1 erfolgt.

4. Verfahren nach einem der Ansprüche 1 bis 3, **dadurch gekennzeichnet, dass** das Abtrennen des Komplexes durch Gefriertrocknung erfolgt.

5. Verfahren nach einem der Ansprüche 1 bis 4, **dadurch gekennzeichnet, dass** das Fluran ausgewählt ist aus der Gruppe bestehend aus Sevofluran, Enfluran, Isofluran, Desfluran und Methoxyfluran.

6. Verfahren nach Anspruch 5, **dadurch gekennzeichnet, dass** das Fluran Sevofluran ist.

7. Komplex aus SBECD und einem Fluran, erhältlich durch ein Verfahren nach einem der Ansprüche 1 bis 6.

8. Komplex nach Anspruch 7, **dadurch gekennzeichnet dass** der Flurangehalt größer als 8 Gew.-%, vorzugsweise größer als 8,2 Gew.-%, weiter vorzugsweise größer als 8,5 Gew.-% ist.

9. Komplex nach Anspruch 7 oder 8, **dadurch gekennzeichnet dass** das Fluran Sevofluran ist.

10. Komplex nach einem der Ansprüche 7 bis 9, **dadurch gekennzeichnet dass** das Molverhältnis SBECD zu Fluran wenigstens 1:0,6, weiter vorzugsweise wenigstens 1:0,7, weiter vorzugsweise wenigstens 1:0,8, weiter vorzugsweise wenigstens 1:0,85 beträgt.

11. Komplex nach einem der Ansprüche 7 bis 10 zur Verwendung als Medikament.

12. Für orale und/oder intravenöse Administration formuliertes Anästhetikum, **dadurch gekennzeichnet, dass** es einen Komplex nach einem der Ansprüche 7 bis 10 aufweist.
